(19) Europäisches Patentamt
European Patent Office
Office européen des brevets

(11) Publication number: 0 336 265 A2

(12) **EUROPEAN PATENT APPLICATION**

(21) Application number: 89105470.2

(51) Int. Cl.4: A61K 7/06

(22) Date of filing: 28.03.89

(30) Priority: 25.03.88 JP 71139/88

(43) Date of publication of application: 11.10.89 Bulletin 89/41

(84) Designated Contracting States: DE FR GB IT

(71) Applicant: AJINOMOTO CO., INC.
5-8, Kyobashi 1-chome, Chuo-ku
Tokyo 104(JP)

(72) Inventor: Nakano, Hiroshi Central Research Laboratories
Ajinomoto Co., Inc No. 1-1, Suzuki-cho
Kawasaki-ku Kawasaki-shi
Kanagawa-ken(JP)
Inventor: Ichiyanagi, Katsuyuki Central Research Lab.
Ajinomoto Co,. Inc No. 1-1, Suzuki-cho
Kawasaki-ku Kawasaki-shi
Kanagawa-ken(JP)

(74) Representative: Strehl, Schübel-Hopf, Groening, Schulz
Maximilianstrasse 54 Postfach 22 14 55
D-8000 München 22(DE)

(54) **Hair cosmetic composition.**

(57) A hair cosmetic composition comprising an $N^{omega}$-mono long chain acyl substituted basic amino acid and a surface active agent is provided, which does not require washing off, has superior hair conditioning qualities, and which in particular shows superior antistatic effect, displays a superior effect of preventing flyaway hair and provides excellent combability. It has further superior storage stability, without separation, coloration and odor, it is safe for the hair and skin, and is well suited for applications such as hair brushing agents, lotions, hair creams, hair liquids, setting lotions and hair sprays.

EP 0 336 265 A2

## HAIR COSMETIC COMPOSITION

The present invention relates to a hair cosmetic composition. In more detail, the present invention relates to a hair cosmetic composition that does not require washing away, has a superior effect of conditioning the hair, causes particularly good antistatic effect and combability, and has a superior effect in preventing flyaway hair at the time of brushing.

In the winter season, or under conditions of low temperature such as in the air conditioned rooms that have become popular in recent years, or when the hair is dried with a hair dryer after washing, static electricity is generated by the friction between the hair and the comb or brush, flyaway hair tends to occur. A further problem is dry hair resulting in breaks and hair loss when the hair undergoes damage by treatment with a comb or brush.

In recent years, the interest in hair care has been increasing, both among men and women, and various hair cosmetics that impart a conditioning effect to the hair, in order to resolve the problems described above, are being marketed and used, in the form of hair rinse, hair treatments and hair creams, and hair brushing agents. Many of these hair cosmetics are blends of cationic surface active agents, or cationized polymers such as cationized cellulose or cationized polyvinyl pyrrolidone derivatives, amphoteric surface active agents, nonionic surface active agents, or oily ingredients such as lanolin and its derivatives, silicones, jojoba oil, and isopropyl myristate, and are ordinarily marketed as lotions, as emulsions in liquid or cream form, or as sprays dissolved in solvents, such as ethanol.

Among the ingredients that are used in these hair care products, cationic surface active agents such as quaternary ammonium salts and pyridinium salts have a very superior conditioning effect compared to others. Many of these cationic surface active agents are, however, strongly stimulating and since there are problems as to their safety, there are restrictions on the amounts that can be mixed in practice, except for hair care products of the washing off type such as hair rinse and hair treatments. In this case they are not capable to display a satisfactory effect. Regarding hair rinses and hair treatments which are hair care products of the washing off type, even those which can be mixed with increased amounts of cationic surface active agents do not display sufficient effect, because the greatest part is completely washed out by rinsing. Consequently, prior hair cosmetics, do not yet show a satisfactory performance of sufficient conditioning of the hair, particularly superior antistatic effect during brushing and superior combability, or superior effect in preventing flyaway hair.

It is therefore the object of the present invention to provide a hair cosmetic composition, which has a superior effect of satisfactory conditioning of the hair, and particularly provides the hair with combability and antistatic effect during brushing, is superior in the effect of preventing flyaway hair, and does not require a washing off operation.

As a result of extensive experiments with the aim to solve these problems it has been found that when an $N^{omega}$-mono long chain acyl derivative of a basic amino acid is combined with a surface active agent, a very superior antistatic effect is shown, even at low concentrations, compared to the case when either compound is used alone, and that this compositon has an excellent conditioning effect that resolves the problems described above. It was further discovered that by adding a macromolecular thickener, or a colloidal moisture-containing silicate, to the above combination, the form stability of the composition will be further improved, and thus the present invention was completed.

The present invention relates to a hair cosmetic composition which is characterized in that it contains, based on the overall amount of the whole composition, (A) 0.2 to 20 wt% of one type or two or more types of an $N^{omega}$-mono long chain acyl basic amino acid whose long chain acyl group is an aliphatic acyl group having 8 to 22 carbon atoms, and (B) 0.02 to 15 wt% of one type or two or more types of a surface active agent, wherein the surface active agent (B) is one or more members selected from anionic surface active agents, cationic surface active agents, amphoteric surface active agents and nonionic surface active agents. This composition has superior effectiveness of preventing flyaway hair during brushing, and does not require a washing off operation. According to a second embodiment this composition is characterized in that it contains, based on the weight of the overall composition, (A) 0.2 to 20 wt% of one type or two or more types of the above defined $N^{omega}$-mono long chain acyl basic amino acid and (B) 0.02 to 15 wt% of one type or two or more types of the above defined surface active agent, and (C) 0.1 to 10 wt% of one type or two or more types of thickener, the said thickener or thickeners (C) being selected from macromolecular thickeners or colloidal water-containing silicates. This composition provides superior hair conditioning of the hair, particularly an antistatic effect and superior combability, and there is a superior effect in preventing flyaway hair during brushing, excellent storage stability and no need for washing off.

As the basic amino acid that is a structural element of the $N^{omega}$-mono long chain acyl basic amino acid, we prefer lysine and ornithine, and these may be either optically active substances or racemic modifications. As the long chain acyl residue (fatty acid residual group), a long chain acyl group (fatty acid residual group) having 8 to 22 carbon atoms can be used, and specifically preferred examples are capryloyl, caproyl, lauroyl, myristoyl, palmitoyl, stearoyl, behenoyl, cocoyl (coconut oil fatty acid acyl), solidified cow's milk fatty acid acyl and cow's milk fatty acid acyl. Mixtures of these acyl groups may also be used.

The $N^{omega}$-mono long chain acyl basic amino acid used in the present invention can be prepared by adding a fatty acid chloride dropwise into an alkaline aqueous solution of the above mentioned basic amino acid. It is possible to obtain it with known methods, such as a method following the so-called Schotten-Baumann reaction, or also by the method of dehydration under heating of a fatty acid salt of basic amino acid, at a temperature from 100° C to 250° C (Japanese Patent Publication Shoo 51-28610). As examples of the $N^{omega}$-mono long chain acyl basic amino acids obtained in this manner, there may be mentioned $N^{epsilon}$-capryloyl lysine, $N^{epsilon}$-lauroyl lysine, $N^{epsilon}$-myristoyl lysine, $N^{epsilon}$-palmitoyl lysine, $N^{epsilon}$-stearoyl lysine, $N^{epsilon}$-behenoyl lysine, $N^{epsilon}$-cocoyl lysine, $N^{epsilon}$-hardened cow's milk fatty acid acyl lysine, $N^{delta}$-caproyl ornithine, $N^{delta}$-lauroyl ornithine, $N^{delta}$-palmitoyl ornithine, $N^{delta}$-stearoyl ornithine, $N^{delta}$-behenoyl ornithine and $N^{delta}$-cow's milk fatty acid acyl ornithine and their mixtures. All of these compounds are insoluble in water and various solvents within the pH ranges ordinarily used in cosmetic products, and are superior in safety, giving no stimuli to humans (Japanese Patent Publication Shoo 61-137812).

On the other hand, it is possible to use any anionic surface active agent, cationic surface active agent, amphoteric surface active agent or nonionic surface active agent as a surface active agent for the purpose of the present invention. One type of surface active agent may be used alone, or two or more types may be used in combination. Examples of these surface active agents are given below.

As anionic surface active agents, there may be used long chain fatty acids salts, alkyl phosphates, polyoxyethylene alkyl ether phosphates, alkyl sulfonates (known as SAS), alpha-olefin sulfonates (known as AOS), monoalkyl sulfosuccinates, dialkyl sulfosuccinates, polyoxyethylene alkyl sulfosuccinates, acyl monoethanolamide polyoxyethylene sulfosuccinate monoester salts, monoalkyl sulfates, polyoxyethylene alkyl ester phosphates, N-methyl-N-alkyl taurine salts, acyl isethionate salts, N-acyl basic amino acids such as N-acyl glutamic acid or N-acyl aspartic acid and their salts, N-acyl neutral amino acids such as N-acyl proline, N-acyl serine, N-acyl sarcosine or N-acyl methyl beta-alanine, and their salts. The carbon numbers of the alkyl groups and acyl groups of these anionic surface active agents are in the range of 8 to 22, and, lithium salt, potassium salt, sodium salt, thriethanol amine salt and basic amino acid salts such as lysine and arginine are preferred among these types of salts.

Examples of cationic surface active agents are compounds such as alkyl trimethyl ammonium salt, dialkyl dimethyl ammonium salt, alkyldimethyl benzyl ammonium salt, alkyl pyridinium salt, dipolyoxyethylene alkyl methyl ammonium salt, tri(polyoxyethylene)alkyl ammonium salt, 2-alkyl-1-alkyl-1-hydroxyethyl imidazolinium salt.

The carbon numbers of the alkyl groups of said cationic surface active agents are 8 to 22, with 16 to 22 being particularly preferred from the standpoint of safety, and the preferred types of salts are those whose counter ions are halogen ions such as chlorine and bromine. Useful cationic surface active agents are furthermore N-long chain acyl basic amino acid alkyl ester salts (wherein the long chain acyl groups have carbon numbers of 8 to 22, the alkyl groups have carbon numbers of 1 to 22, the basic amino acid residual groups are such as arginine, lysine and ornithine, the types of salts are hydrochlorides and pyrrolidone carbonates), and $N^{omega}$-long chain acyl-$N^{alpha}$, $N^{alpha}$, $N^{alpha}$-trimethyl basic amino acid (with such as lysine and ornithine as the basic amino acid).

As examples of the amphoteric surface active agents, may be mentioned alkyl dimethylamino betaine acetate, 2-alkyl-N-carboxymethyl-N-hydroxyethyl imidazolinium betaine, higher fatty acid amidopropyl dimethylamino betaine acetate, N-alkyl-N, N-dimethyl-N-sulfoalkylene ammonium betaine, and $N^{omega}$-mono long chain acyl-$N^{alpha}$ and $N^{alpha}$-dimethyl lysine. Further, the carbon numbers of the alkyl groups and acyl groups (fatty acid residual groups) are 8 to 22.

Among nonionic surface active agents are compounds such as polyoxyethylene alkyl ether, polyoxyethylene cholesteryl ether, polyoxyethylene sorbitan fatty acid partial ester, polyoxyethylene sorbitol fatty acid partial ester, polyoxyethylene glycerin fatty acid ester, polyoxyethylene hardened castor oil, polyoxyethylene hardened castor oil fatty acid ester, polyglycerin fatty acid ester, cane sugar fatty acid ester, N-acyl glutamic acid polyoxyethylene alkyl ether diester, monopyroglutamic acid mono fatty acid polyoxyethylene hardened castor oil, monopyroglutamic acid mono fatty acid polyoxyethylene glycerin, monopyroglutamic acid mono fatty acid glycerin, mono fatty acid ethylene glycol, mono fatty acid propylene glycol, sorbitan fatty acid partial ester, glycerin fatty acid partial ester, fatty acid alkylol amide and alkylamine oxide.
The carbon numbers of alkyl groups and acyl groups (fatty acid residual groups) are 8 to 22.

The hair cosmetic composition of the present invention that does not require a washing off operation, has $N^{omega}$-mono long chain acyl basic amino acid and a surface active agent as indispensable components.

It is necessary that the mixture amount of $N^{omega}$-mono long chain acyl basic amino acid be at least 0.02 wt%, preferably at least 0.2 wt%, below which its properties are difficult to display. More preferably the mixture amount should be at least 0.1 wt%. On the other hand, while there are no particular restrictions as to its upper limit, this will preferably be 20% or less from the standpoint of product form and economy. Also, it is necessary that in the mixture the amount of the surface active agent be at least 0.02 wt%, below which its properties are difficult to display, and preferably the added amount should be at least 0.1 wt%. On the other hand, while there are no particular restrictions as to its upper limit, this will preferably be 15 wt% or less from considerations of product form and economy, and action on the hair and skin.

For the thickener used in the second embodiment of the present invention, examples are macromolecular thickeners such as carboxy vinyl polymers, sodium polyacrylate, sodium alginate, propylene glycol alginate, carboxymethylcellulo sodium, methylcellulose, ethylcellulose, hydroxyethylcellulose, hydroxypropylcellulose, polyethylene glycol distearate and polyoxyethylene(120)-methyl glucose dioleate, and colloidal moisture-containing silicates such as colloidal moisture-containing aluminum silicate (bentonite) and colloidal moisture-containing aluminum magnesium silicate. The amount of the thickener in the mixture must be at leaset 0.1%, below which it is difficult to improve storage stability. On the other hand, while there are no particular restrictions on the upper limit of the mixture amount, it is preferred that this be 10% or less from the standpoint of product form and economy.

The form of the composition of the present invention can be any form such as liquid, emulsion or cream, or powder, and may also be of the liquid or nonliquid type. The composition of the present invention can be manufactured easily according to various conventional methods known in this specific field, depending on its form. It will display a conditioning effect on the hair with any of these methods, such as methods of applying directly on the hair by hand, or with a comb or a brush, or methods of packing it in a pressure container or aerosol type container and blowing it onto the hair as a misty spray or a foamed mass.

In order to carry out in practice the above described forms and methods of use with the composition of the present invention, and in order to raise the value of the product, it is possible to incorporate, according to the need, various known ingredients such as solvents, oleophilic ingredients (oily ingredients), hydrophilic ingrendients (aqueous ingredients) and macromolecular compounds that have been in prior use for cosmetic applications, and examples of these ingredients are give below.

As dispersing media and solvent media, there may be used solvents such as water, ethanol and isopropanol, and as blowing or jetting agents, there may be mentioned Freon gas, liquefied petroleum gas and ether type gases such as dimethyl ether.

Among useful oleophilic ingredients, are hydrocarbons such as fluid paraffin, squalane, vaseline and microcrystalline wax, fats and oils such as olive oil, coconut oil, camellia oil, Japan wax and castor oil, waxes such as beeswax, whale wax, hohoba oil, lanolin and caranauba wax, higher fatty acids such as lauric acid, myristic acid, palmitic acid, stearic acid, isostearic acid, olenic acid and lanolin fatty acid, higher alcohols such as lauryl alcohol, myristyl alcohol, cetanol, stearyl alcohol, cetostearyl alcohol, oleyl alcohol, cholesterol, lanolin alcohol and octyl dodecanol, esters such as tri(capryl caprynic acid) glycerin, hexyl laurate, glycerin trilaurate, isopropyl myristate, octyldodecyl myristate, cetyl myristate, myristyl myristate, glycerin trimyristate, isopropyl palmitate, butyl stearate, octyldodecyl oleate, decyl oleate, N-acyl glutamate higher alcohol ester and hardened oils, and silicones such as methylpolysiloxane, methylphenylpolysiloxane and cyclic silicones.

As the hydrophilic ingredient, there may be mentioned, acids, bases and their salts or such compounds as glycerin, propylene glycol, 1,3-butylene glycol, Sorbit, polyethylene glycol, hexylene glycol, hydrolyzed protein, amino acid, dl-pyrrolidone sodium carbonate, wetting agents such as sodium lactate, citric acid, tartaric acid, dl-pyrrolidone carbonic acid, lactic acid, phosphoric acid, borax, sodium hydroxide, potassium hydroxide and triethanolamine.

Examples of the macromolecular compounds, are coat forming agents such as polyvinyl alcohol, polyvinyl acetate, tragacanth gum, shellac, methoxyethylene-maleic anhydride copolymer, polyvinyl pyrrolidone, polyvinyl pyrrolidone vinyl acetate copolymer, polyvinyl pyrrolidone dimethyl aminoethyl methacrylic acid copolymer, cationized cellulose and polyoxypropylene butyl ether.

Further, if required the composition of the present invention may contain various ingredients in prior use in the field of cosmetic products, other than the ingredients described above, particularly such as colorants, perfumes, preservatives, pasteurizers, oxidation inhibitors, ultraviolet ray absorbers, inorganic powders, or pharmaceutically effective ingredients such as itching inhibitors, hair growing agents and vitamins.

The hair cosmetic composition that does not require washing off, according to the present invention, has superior hair conditioning qualities, and in particular confers to the hair superior antistatic effect and combability, and has a characterizing feature in being superior in the effect of prevent flyawy hair. These effects constitute qualities that could not be satisfactorily achieved in prior products that were combinations of cationic surface active agents, conditioning agents and lubricating powders. The present composition is also superior in storage stability, without separation, coloration and odor, it has good safety to the hair and skin, and it is well suited for applications, such as hair brushing agents, lotions (hair tonics), hair creams, hair liquids, setting lotions and hair sprays.

Thereafter examples of the present invention will be given; it should of course be understood that the present invention is not limited thereby.

Further, before explaining the examples, we will explain below the test methods for hair conditioning properties and storage stability adopted for the examples.

Hair conditioning properties were measured or evaluated under the four headings of antistatic effect, flyaway prevention effect, combability and smoothness.

The following Reference Literature was used:

(1) Kazutami Sakamoto, Makiko Tanaka, Noriko Ide, Hiroshi Yokota, Masahiro Takehara, "Moohatsu taiden tokusei sokutei-ki no kaihatsu to ooyoo [Development and application of instruments for measuring hair static electric properties]," Nihon Kooshoohin Kagakkai Shi [Journal of the Japanese Perfume and Cosmetic Science Society], 8, 330 (1984).

The hair used for treatment was as follows:

## Hair Used in Tests

We used the hair of five women, and prepared it based on the above Reference Literature (1), as hair pieced 11 cm long, 4 cm wide and 4 g in weight, arranged in the cuticle direction.

## Test Method (1) : Antistatic Effect

This was done following Reference Literature (1). That is, as a pretreatment, the hair used for testing was repeatedly cleansed with an aqueous solution of polyoxyethylene lauryl ether sodium sulfate and washed with water, and was dried by letting it stand for 24 hours in a constant temperature chamber at 25°C, 40% RH. Then 0.1 g of the sample was directly applied on, or blown on, uniformly spread with a comb, and again dried in the said constant temperature chamber for 24 hours. After drying, static electricity was generated by combing with an aluminum comb electrode, and the amount of static electricity was measured with a universal electrometer (made by Kawaguchi Denki Seisakusho K.K. [Kawaguchi Electric Works Co., Ltd.], Model MMAII-17). For the evaluation of the antistatic effect, since the amount of static electricity is sensitive to humidity, the quantiy (Q) of static electricity of the hair treated with the sample was evaluated using as reference the quantity (q) of the static electrictiy of the hair used only in the pretreatment test. Both hair samples were simultaneously measured and evaluated according to the four stages below as the ratio (Q/q).

| | | |
|---|---|---|
| ◎ | Very superior | (1/30 or less) |
| O | Superior | 1/10 or less) |
| Δ | Some effect present | (1/3 or less) |
| X | No effect | (over 1/3) |

## Test Method (2): Flyaway Prevention Effect

The pretreatment and test treatment of the test hair were done under conditions identical to Test Method (1), and after drying, it was brushed 10 times with a nylon brush, and the expansion of hair (flyaway) at this time was measured in the wide direction at the lower end of the test hair (unit cm). When the expansion was 6 cm or less, it was said to have good flyaway prevention effect.

Test Method (3): Combability, Smoothness

The pretreatment and test treatment of the test hair were done under conditions identical to Test Method (1), and after drying, it was given sensory evaluation as to combability and smoothness. The evaluations were in four stages, based on the test hair in pretreatment only.

| | |
|---|---|
| ◎ | Very superior |
| O | Superior |
| Δ | Rather good |
| X | No difference |

Storage Stability Test

Compositions were stored at 40 °C and observed for one month. Those in which no separation or coloration were observed with the naked eye, and where no odor was found, were designated as O, and those where any of these were found were designated as X.

Further, in each table the amount of the constituents is indicated as wt%.

Example 1

Hair cosmetic compositions were compounded of various constituents as shown in Table 1, Table 2, Table 3 and Table 4, and their performances were evaluated. The results are shown in the same tables.

Table 1.

Table 1.

| | Example 1: Test No. | 1 | 2 | 3 | 4 | 5 | 6 | 7 | 8 | 9 | 10 | 11 | | | | | | | | | |
|---|---|---|---|---|---|---|---|---|---|---|---|---|---|---|---|---|---|---|---|---|---|
| | Comparative Example: Test No. | | | | | | | | | | | | 1 | 2 | 3 | 4 | 5 | 6 | 7 | 8 | 9 |
| Composition | $N^{epsilon}$-myristoyl lysine | 0.5 | 0.5 | 0.5 | 0.5 | 0.5 | 0.5 | 0.5 | 0.5 | 0.5 | 0.5 | 0.5 | | | | | | | | | |
| | POE (2) lauryl ether triethanolamine phosphate | 0.5 | | | | | | | | | | | 0.5 | | | | | | | | |
| | alpha-Olefin sodium sulfate | | 0.5 | | | | | | | | | | | 0.5 | | | | | | | |
| | $N^{alpha}$-myristoyl potassium glutamate | | | 0.5 | | | | | | | | | | | 0.5 | | | | | | |
| | Monolauryl arginine sulfate | | | | 0.5 | | | | | | | | | | | 0.5 | | | | | |
| | Cetyl trimethyl ammonium bromide | | | | | 0.5 | | | | | | | | | | | 0.5 | | | | |
| | $N^{alpha}$-cocoyl arginine ethyl ester dipyrrolidone carbonate salt | | | | | | 0.5 | | | | | | | | | | | 0.5 | | | |
| | Cocoamidopropyl betaine | | | | | | | 0.5 | | | | | | | | | | | 0.5 | | |
| | POE (30) cetyl ether | | | | | | | | 0.5 | | | | | | | | | | | 0.5 | |
| | POE (20) sorbitan monolaurate | | | | | | | | | 0.5 | | | | | | | | | | | 0.5 |
| | POE (40) glycerin monostearate | | | | | | | | | | 0.5 | | | | | | | | | | |
| | Cane sugar fatty acid ester | | | | | | | | | | | 0.5 | | | | | | | | | |
| | Purified water | 99.0 | 99.0 | 99.0 | 99.0 | 99.0 | 99.0 | 99.0 | 99.0 | 99.0 | 99.0 | 99.0 | 99.5 | 99.5 | 99.5 | 99.5 | 99.5 | 99.5 | 99.5 | 99.5 | 99.5 |
| Performance | Antistatic effect | ◎ | ◎ | ◎ | ◎ | ◎ | ◎ | ◎ | ◎ | ◎ | ◎ | ◎ | × | × | × | × | △ | △ | × | × | × |
| | Flyaway prevention effect (cm) | 5 | 5 | 5 | 5 | 4 | 4 | 4 | 5 | 5 | 5 | 5 | 10 | 10 | 8 | 9 | 7 | 7 | 8 | 9 | 8 |
| | Combability | ◎ | ◎ | ◎ | ◎ | ◎ | ◎ | ◎ | ◎ | ◎ | ◎ | ◎ | × | × | × | × | △ | △ | × | × | × |
| | Smoothness | ◎ | ◎ | ◎ | ◎ | ◎ | ◎ | ◎ | ◎ | ◎ | ◎ | ◎ | × | × | △ | × | △ | △ | △ | × | × |

(Note) POE is an abbreviation for polyoxyethylene.

Table 2.

| | | 12 | 13 | 14 | 15 | 16 | 17 | 18 | 19 | | | 20 | 21 | 22 | 23 | 24 | 25 |
|---|---|---|---|---|---|---|---|---|---|---|---|---|---|---|---|---|---|
| | Example 2: Test No. | 12 | 13 | 14 | 15 | 16 | 17 | 18 | 19 | | | 20 | 21 | 22 | 23 | 24 | 25 |
| | Comparative Example: Test No. | | | | | | | | | 12 | 13 | | | | | | |
| Composition | $N^{epsilon}$-capryloyl lysine | 0.5 | | | | | | | | | | | | | | 0.1 | |
| | $N^{epsilon}$-lauroyl lysine | | | | | | | | | | | 0.2 | 0.2 | | | 0.1 | |
| | $N^{epsilon}$-stearoyl lysine | | 0.5 | | | | | | 2.0 | 2.0 | | | | 0.1 | | | |
| | $N^{epsilon}$-cocoyl lysine | | | 0.5 | | | | | | | | | | | | | 0.5 |
| | $N^{epsilon}$-hardened hardened cow's milk fatty acid acyl lysine | | | | 0.5 | | | | | | | | | | 0.5 | | |
| | $N^{epsilon}$-caproyl ornithine | | | | | 0.5 | | | | | | | | | 0.5 | 0.1 | |
| | $N^{epsilon}$-myristoyl ornithine | | | | | | 0.5 | | | | | | | | | | 0.5 |
| | $N^{epsilon}$-behenoyl ornithine | | | | | | | 0.5 | | | | | | 0.1 | | | |
| | POE (2) lauryl ether sodium sulfate | 0.5 | 0.5 | 0.5 | 0.5 | 0.5 | 0.5 | 0.5 | | | | 0.1 | 0.1 | | | | |
| | POE (2) lauryl ether potassium sulfate | | | | | | | | | | | | | | | 0.2 | |
| | N-cocoyl triethanolamine glutamate | | | | | | | | | | | | | | | | 0.5 |
| | Stearyl dimethylbenzyl ammonium chloride | | | | | | | | 1.0 | | 1.0 | | | | | | |
| | POE (5) cholesteryl ether | | | | | | | | | | | | | | 0.5 | | |
| | POE (40) hardened castor oil | | | | | | | | | | | 0.2 | 0.2 | | | | |
| | Lipophilic glycerin monostearate | | | | | | | | | | | | | 0.2 | | | |
| | Ethanol | | | | | | | | 97.0 | 98.0 | 99.0 | 99.5 | | 99.6 | 98.5 | | |
| | Purified water | 99.0 | 99.0 | 99.0 | 99.0 | 99.0 | 99.0 | 99.0 | | | | | 99.5 | | | 99.5 | 98.5 |
| Performance | Antistatic effect | ◎ | ◎ | ○ | ◎ | ○ | ◎ | ◎ | ○ | × | △ | ◎ | ○ | ○ | ○ | ◎ | ○ |
| | Flyaway prevention effect (cm) | 5 | 5 | 4 | 5 | 4 | 5 | 5 | 4 | 9 | 7 | 5 | 5 | 5 | 4 | 5 | 4 |
| | Combability | ◎ | ◎ | ○ | ◎ | ○ | ◎ | ◎ | ○ | × | △ | ○ | ○ | ◎ | ○ | ○ | ○ |
| | Smoothness | ○ | ◎ | ○ | ◎ | ○ | ◎ | ◎ | ○ | × | △ | ◎ | ○ | ○ | ○ | ◎ | ○ |

(Note) POE is an abbreviation for polyoxyethylene.

Table 3.

| | | 26 | 27 | | 28 | | 29 | | 30 | | 31 | | 32 | | 33 | | 34 | |
|---|---|---|---|---|---|---|---|---|---|---|---|---|---|---|---|---|---|---|
| | Example 3: Test No. | 26 | 27 | | 28 | | 29 | | 30 | | 31 | | 32 | | 33 | | 34 | |
| | Comparative Example: Test No. | | | 14 | | 15 | | 16 | | 17 | | 18 | | 19 | | 20 | | 21 |
| Composition | $N^{epsilon}$-caproyl lysine | 20.0 | 0.02 | 0.01 | 0.20 | 0.20 | | | | | | | | | | | | |
| | $N^{epsilon}$-behenoyl lysine | | | | | | 0.02 | 0.01 | 0.20 | 0.20 | | | | | 0.02 | 0.01 | 0.20 | 0.20 |
| | $N^{epsilon}$-cow's milk fatty acid acyl lysine | | | | | | | | | | 0.02 | 0.01 | 0.20 | 0.20 | | | | |
| | $N^{epsilon}$-lauroyl ornithine | | | | | | | | | | | | | | | | | |
| | POE (2) lauryl potassium NIITERURINate | 15.0 | 0.20 | 0.20 | 0.02 | 0.01 | | | | | | | | | | | | |
| | Stearyl dimethylbenzyl ammonium chloride | | | | | | 0.20 | 0.20 | 0.02 | 0.01 | | | | | | | | |
| | 2-Alkyl-N-carboxymethyl-N-hydroxyethylimadazolium betain | | | | | | | | | | | | | | 0.20 | 0.20 | 0.02 | 0.01 |
| | POE (5) cholesteryl ether | | | | | | | | | | 0.20 | 0.20 | 0.02 | 0.01 | | | | |
| | Ethanol | | | | | | | | | | 99.78 | 99.79 | 99.78 | 99.79 | | | | |
| | Purified water | 65.0 | 99.78 | 99.79 | 99.78 | 99.79 | 99.78 | 99.79 | 99.78 | 99.79 | | | | | 99.78 | 99.79 | 99.78 | 99.79 |
| Performance | Antistatic effect | O | O | × | O | × | O | × | O | × | O | × | O | × | O | × | O | × |
| | Flyaway prevention effect (cm) | 4 | 6 | 9 | 6 | 8 | 5 | 8 | 6 | 9 | 6 | 10 | 6 | 9 | 6 | 10 | 6 | 9 |
| | Combability | O | O | × | O | × | O | × | O | × | O | × | O | × | O | × | O | × |
| | Smoothness | O | O | × | O | × | O | × | O | × | O | × | O | × | O | × | O | × |

(Note) POE is an abbreviation for polyoxyethylene.

Table 4.

| | | | | | | | | | | | | |
|---|---|---|---|---|---|---|---|---|---|---|---|---|
| | Example 4: Test No. | 35 | 36 | 37 | 38 | 39 | | 40 | 41 | | 42 | 43 |
| | Comparative Example: Test No. | | | | | | 22 | | | 23 | | |
| Composition | $N^{epsilon}$-cocoyl lysine | 2.0 | | | | | | | | | | |
| | $N^{epsilon}$-stearoyl lysine | | 2.0 | | 2.0 | | | | | 1.0 | 1.0 | 1.0 |
| | $N^{delta}$-lauroyl ornithine | | | 2.0 | | 2.0 | 4.0 | 4.0 | 4.0 | | | |
| | POE (2) lauryl ether sodium sulfate | | | | 2.0 | | | | | | | |
| | Stearylpentaethoxy ammonium chloride | | | 2.0 | | | | | | | | |
| | Lauryl diaminoacetate betaine | 2.0 | | | | | | | | 1.0 | 1.0 | 1.0 |
| | POE (20) oleyl ether | | 2.0 | | | | 2.0 | 2.0 | 2.0 | | | |
| | Pentaglycerin distearate | | | | | 2.0 | | | | | | |
| | Colloidal moisture-containing aluminum magnesium silicate | 1.0 | | | | | | | | | | |
| | Sodium polyacrylate | | 1.0 | | | | 0.05 | 0.1 | 10.0 | | | |
| | Hydroxyethylcellulose | | | 1.0 | | | | | | | | |
| | Propylene glycol arginate | | | | 1.0 | | | | | | | |
| | Hydroxypropylcellulose | | | | | 1.0 | | | | 0.05 | 0.1 | 10.0 |
| | Ethanol | | | | 20.0 | 95.0 | | | | 97.95 | 97.9 | 88.0 |
| | Purified water | 95.0 | 95.0 | 95.0 | 75.0 | | 93.95 | 93.9 | 84.0 | | | |
| Performance | Antistatic effect | ◎ | ◎ | ◎ | ◎ | ◎ | ◎ | ◎ | ◎ | ◎ | ◎ | ◎ |
| | Flyaway prevention effect (cm) | 5 | 5 | 4 | 5 | 4 | 5 | 5 | 5 | 4 | 4 | 4 |
| | Combability | ◎ | ◎ | ◎ | ◎ | ◎ | ◎ | ◎ | ○ | ◎ | ◎ | ○ |
| | Smoothness | ◎ | ◎ | ◎ | ◎ | ◎ | ◎ | ◎ | ○ | ◎ | ◎ | ○ |
| | Storage stability | ○ | ○ | ○ | ○ | ○ | × | ○ | ○ | × | ○ | ○ |

(Note) POE is an abbreviation for polyoxyethylene.

Example 2

Hair conditioning compositions having the constituents shown in Table 5, Table 6 and Table 7 were prepared based on the present invention, and their performances were evaluated.

Table 5

| Hair Brushing Agent Composition (Aqueous System). | |
|---|---|
| Ingredients | Mixture Amount, wt% |
| $N^{epsilon}$-myristoyl lysine | 0.5 |
| Monolauric acid POE (50) sorbitan | 0.4 |
| Monopyroglutamic acid monoisostearic acid | 0.4 |
| Carboxy vinyl polymer | 2.0 |
| Preservative (methylparaben) | 0.2 |
| Perfume | 0.2 |
| Purified water | Balance to 100 wt% |

Table 6

| Hair Brushing Agent Composition (Nonaqueous System). | |
|---|---|
| Ingredients | Mixture Amount, wt% |
| $N^{delta}$-lauroyl ornithine | 0.5 |
| Distearyl chloride dimethyl ammonium | 0.1 |
| Monopyroglutamic glycerin monooleate | 0.5 |
| Dimethylpolysiloxane | 0.2 |
| Perfume | 0.2 |
| Hydroxypropylcellulose | 2.0 |
| Ethanol | Balance |

Table 7

| Hair Brushing Agent (Foam). | |
|---|---|
| Ingredients | Mixture Amount, wt% |
| $N^{epsilon}$-cocoyl lysine | 2.0 |
| N-cocoyl potassium glutamate | 1.0 |
| Palm oil fatty acid diethanolamide | 1.0 |
| POE (50) hardened castor oil | 2.0 |
| Octyldodecanol | 0.5 |
| Squalene | 0.5 |
| Preservative (methylparaben) | 0.1 |
| Perfume | 0.2 |
| Ethanol | 5.0 |
| Purified water | 40.0 |
| Jetting agent (liquefied natural gas) | Balance |

These hair brushing compositions were superior in antistatic prevention performance at time of brushing and in combability, and because of the fact that they strongly showed prevention of flyaway, they were effective in preventing branching hair and split hair.

Example 3

Hair lotion (hair tonic) compositions having the constituents shown in Table 8 and Table 9 were prepared based on the present invention, and their performances were evaluated.

Table 8

| Hair Lotion (Conditioning Type). | |
|---|---|
| Ingredients | Mixture Amount, wt% |
| $N^{delta}$-behenoyl ornithine | 0.5 |
| $N^{epsilon}$-caproyl lysine | 0.5 |
| Stearyl alkyl dimethylamine oxide | 0.5 |
| Monopyroglutamic acid POE (30) glycerin monoisostearate | 1.5 |
| Hohoba oil | 0.1 |
| 1,3-butylene glycol | 3.0 |
| Arginic acid propylene glycol | 0.5 |
| Preservative (methylparaben) | 0.1 |
| Ethanol | 2.0 |
| Purified water | Balance |

Table 9.

| Hair Lotion (Tonic Type). | |
|---|---|
| Ingredients | Mixture Amount, wt% |
| $N^{epsilon}$-lauroyl lysine | 0.1 |
| $N^{alpha}$-myristoyl arginine ethyl ester hydrochloride | 0.1 |
| POE (10) cholesteryl ether | 0.2 |
| Menthol | 0.1 |
| Glycerin | 3.0 |
| Hydroxy propylcellulose | 0.5 |
| Perfume | Suitable amount |
| Colorant | Suitable amount |
| Ethanol | Balance |

These hair lotion compositions were superior in antistatic prevention performance at time of brushing and in combability, and because of the fact that they markedly prevented flyaway, they were effective in preventing branching hair and split hair.

Example 4

A hair cream composition having the constituents shown in Table 10 was prepared based on the present invention, and its performance was evaluated.

Table 10.

| Hair Cream Composition. | |
|---|---|
| Ingredients | Mixture Amount, wt% |
| Fluid paraffin | 10.0 |
| Liquid lanolin | 2.0 |
| Cetanol | 4.0 |
| N-delta-stearoyl ornithine | 5.0 |
| Self-emulsifying type glycerin monostearate | 2.0 |
| POE (20) glycerin monostearate | 2.0 |
| N-stearoyl sodium glutamate | 0.2 |
| L-proline | 0.3 |
| Glycerin | 5.0 |
| Preservative | Suitable amount |
| Purified water | Balance |

This hair cream composition was superior in antistatic prevention performance at time of brushing and in combability, and it was effective in preventing branching hair and split hair.

Example 5

A hair liquid composition having the constituents shown in Table 11 was prepared based on the present invention, and its performance was evaluated.

Table 11.

| Hair Liquid Composition. | |
|---|---|
| Ingredients | Mixture Amount, wt% |
| Polyoxypropylene (40) butyl ether | 20.0 |
| Nepsilon-stearoyl lysine | 0.5 |
| Cocoamidopropyl betaine | 0.5 |
| POE (60) hardened castor oil | 1.0 |
| Propylene glycol | 3.0 |
| Propynyl pyrrolidine | 1.0 |
| Isopropanol | 3.0 |
| Ethanol | 50.0 |
| Perfume | Suitable amount |
| Purified water | Balance |

This hair liquid composition was superior in antistatic prevention performance at time of brushing and in combability, and it was effective in preventing branching hair and split hair.

Example 6

A setting lotion composition having the constituents shown in Table 12 was prepared based on the present invention, and its performance was evaluated.

Table 12.

| Set Lotion Composition. | |
|---|---|
| Ingredients | Mixture Amount, wt% |
| Polyvinyl pyrrolidone | 3.0 |
| Poly(vinyl pyrrolidone/vinyl acetate)copolymer | 0.5 |
| $N^{delta}$-myristoyl ornithine | 0.3 |
| Pentaglycerine monostearate | 0.5 |
| POE (60) monostearate | 0.5 |
| Polyethylene glycol 400 | 2.0 |
| Ethanol | 30.0 |
| Preservative | Suitable amount |
| Perfume | Suitable amount |
| Purified water | Balance |

This set lotion composition was superior in antistatic prevention performance at time of brushing and in combability, and since it markedly prevented flyaway, it was effective in preventing branching hair and split hair.

Example 7

A hair spray composition having the constituents shown in Table 13 was prepared based on the present invention, and its performance was evaluated.

Table 13.

| Hair Spray Composition. | |
|---|---|
| Ingredients | Mixture Amount, wt% |
| Poly(vinyl pyrrolidone/vinyl acetate)copolymer | 2.5 |
| Methyl phenyl polysiloxane | 0.1 |
| $N^{epsilon}$-hardened cow's milk fatty acid acyl lysine | 0.5 |
| PE (20) cholesteryl ether | 0.4 |
| Ethanol | 35.0 |
| Perfume | Suitable amount |
| Jetting agent (Freon gas) | Balance |

This hair spray composition was superior in antistatic prevention performance at time of brushing and in combability, and since it markedly prevented flyaway, it was effective in preventing branching hair and split hair.

## Claims

1. Hair cosmetic composition containing (A) 0.2 to 20 wt% of one type or two or more types of $N^{omega}$-mono long chain acyl substituted basic amino acid wherein the long chain acyl group is an aliphatic acyl group having 8 to 20 carbon atoms, and (B) 0.02 to 15 wt% of one type or two or more types of surface active agent, and optionally (C) 0.1 to 10 wt% of one type or two or more types of thickener.

2. Hair cosmetic composition according to claim 1 wherein the surface active agent is selected from anionic surface active agents, cationic surface active agents, amphoteric surface active agents or nonionic surface active agents.

3. Hair cosmetic composition according to Claim 1 or 2 wherein the thickener is a macromolecular thickener or a colloidal moisture-containing silicate.